# EUROPEAN PATENT APPLICATION

(11) **EP 1 450 156 A1**
(43) Date of publication of application: **25.08.2004**
(21) Application number: 03746449.2
(22) Date of filing: 10.04.2003
(51) Int. Cl.: G01N 27/22

(54) **SENSOR CELL, BIOSENSOR, CAPACITIVE DEVICE MANUFACTURING METHOD, BIOLOGICAL REACTION DETECTION METHOD, AND GENE ANALYZING METHOD**

(30) Priority: 12.04.2002 JP 2002110822
(71) Applicant: Seiko Epson Corporation, Shinjuku-ku, Tokyo 163-0023 (JP)
(72) Inventor: MAEDA, Hiroshi, SEIKO EPSON CORPORATION, Suwa-shi, Nagano 392-8502 (JP)
(74) Representative: Kenyon, Sarah Elizabeth
(86) International application number: PCT/JP2003/004576
(87) International publication number: WO 2003/087798

(57) **Abstract**

The present invention provides a bio-sensor comprising a sensor cell matrix in which sensor cells are arranged into a matrix, a row driver which supplies a specific voltage signal to a group of sensor cells lined up in the row direction of the matrix, and a column driver which supplies a specific voltage signal to a group of sensor cells lined up in the column direction of the matrix. Each sensor cell comprises a capacitance element Cs consisting of a pair of opposing electrodes with probe DNAs that react selectively with target DNAs immobilized to their surfaces, a transistor Tr2 whose gate terminal is connected to the capacitance element Cs so that the current value that is output from the drain terminal of this transistor is caused to vary in accordance with the amount of the capacitance variation of the capacitance element Cs which is varied by the hybridization of the DNA, and a switching element Tr1 which supplies a voltage signal supplied from the column driver to the current input terminal of the transistor Tr1.

## Description

### BACKGROUND OF THE INVENTION

### 1. Field of the Invention

The present invention relates to a bio-sensor used in genetic analysis, analysis of biological information and the like.

### 2. Description of the Related Art

In recent years, as a result of progress in the genome project, the genetic structures of various living organisms have gradually become clear, and the reading of DNA base sequences and the functional analysis of genetic information have also become tasks for the purpose of linking these results to life phenomena. DNA micro-arrays have been utilized as systems for monitoring the expression of all genes in a cell at one time. In such arrays, probe DNAs are prepared by performing a reverse transcription reaction of mRNAs or total RNA extracted from cells or tissues, and they are stamped at a high density on a substrate such as a slide glass or the like. Then, among the target DNAs labeled with a fluorescent dye, the target DNAs having complementary base sequences to those of the probe DNAs hybridize with the probe DNAs, and the amount of genetic expression is evaluated by observing the fluorescence pattern.

However, in the case of said DNA micro-arrays, a fluorescent reaction or the like is detected by optical means; as a result, the apparatus is large, and it is difficult to detect the hybridization in real time. Similarly, in case of automatic sequencing by the Sanger method, DNA fragments labeled with a fluorescent dye are separated by gel electrophoresis; then, the dye in the labeled fragments is excited by irradiation with laser light, and the resulting signals are detected by means of a fluorescent light detector. As a result, the apparatus is large, and monitoring cannot be performed in real time.

Accordingly, it is an object of the present invention to provide a technique which makes it possible to analyze large quantities of genetic information in real time using a simple construction.

### SUMMARY OF THE INVENTION

In order to solve the abovementioned problems, the sensor cell of the present invention comprises a capacitance element which consists of a pair of opposing electrodes having receptors immobilized to their surfaces, wherein said receptors are biologically recognizable molecules that react selectively with specific biomolecules, and a transducer which outputs as an electrical signal the amount of the capacitance variation of said capacitance element, wherein said capacitance variation is caused by the reaction between said receptors and biomolecules. As a result of such a structure, biological reactions can be detected as electrical signal; accordingly, the detection signal from the sensor cell can be converted into numerical values and can be subjected to data processing by a computer, so that this sensor cell is suitable for large-scale genetic analysis.

Preferably, each of said pair of opposing electrodes is formed as comb electrode that is formed into a comb shape, or is formed as electrode which has a plurality of circular-arc-form electrode parts with different internal diameters, and forms a circular-arc-form capacitor between the facing electrode parts. As a result of such a structure, the capacitance of the capacitor can be increased, so that the sensitivity of the sensor can be improved.

Preferably, an insulating film is formed between said pair of opposing electrodes to partition the respective electrodes. As a result of such a structure, the distance between the electrodes of the capacitance element can be shortened, and the capacitance of the capacitor can be increased, so that the sensitivity of the sensor can be improved.

Preferably, fine metal particles are deposited on the surfaces of said electrodes. As a result of such a structure, the distance between the electrodes of the capacitance element can be shortened, and the capacitance of the capacitor can be increased, so that the sensitivity of the sensor can be improved.

Preferably, the material of said fine metal particles is a material selected from a group consisting of gold, silver, platinum and copper. Immobilization of the probe DNAs is facilitated by the utilization of such materials.

Preferably, said pair of opposing electrodes is formed inside a reaction well. As a result of such a structure, biological reactions inside the extremely small-volume reaction wells formed in each sensor cell can be detected, so that the sample volume can be extremely small, which is economical.

Preferably, said transducer is a field effect transistor which varies the mutual conductance in accordance with the capacitance variation of said capacitance element. By using a field effect transistor, it is possible to detect the capacitance variation of the capacitance element as the drain current variation.

Preferably, said biologically recognizable molecules are probe DNAs. As a result, a DNA micro-sensor array that can detect DNA hybridization in real time can be realized.

The bio-sensor of the present invention comprises a sensor cell matrix in which sensor cells that output biological reactions as electrical signals are arranged into a matrix, a row driver which selectively supplies a specific voltage signal to row-selecting lines connected to groups of sensor cells that are lined up in the row direction of said sensor cell matrix, and a column driver which supplies a specific voltage signal to column-selecting lines connected to groups of sensor cells that are lined up in the column direction of said sensor cell matrix. Each of said sensor cells comprises a capacitance element which consists of a pair of opposing electrodes having receptors immobilized to their surfaces, wherein said receptors are biologically recognizable molecules that react selectively with specific biomolecules, a transistor whose current-controlling terminal is connected to said capacitance element, so that this transistor varies the current value that is output from the current output terminal in accordance with the amount of the capacitance variation of said capacitance element, wherein said capacitance variation is caused by the reaction between said receptors and biomolecules, and a switching element which supplies the voltage signal supplied from the column driver to the current input terminal of the transistor, In such a structure, said switching element is placed in an open state by the voltage signal supplied from the row driver via said row-selecting line, and the voltage signal supplied from the column driver via said column-selecting line is input into the current input terminal of said transistor. As a result of such a structure, biological reactions can be detected as electrical signals; accordingly, the detection signals from the sensor cell can be converted into numerical values, and can be subjected to data processing by a computer, so that this sensor cell is suitable for large-scale genetic analysis.

Preferably, each of said pair of opposing electrodes is formed as comb electrode that is formed into a comb shape, or is formed as electrode which has a plurality of circular-arc-form electrode parts with different internal diameters, and forms a circular-arc-form capacitor between the facing electrode parts. As a result of such a structure, the capacitance of the capacitor can be increased, so that the sensitivity of the sensor can be improved.

The capacitance element manufacturing method of the present invention is a method for manufacturing a capacitance element whose capacitance is varied by biological reactions; this method comprises the steps of forming a pair of opposing electrodes on the surface of an insulating substrate, coating the surfaces of said opposing electrodes with fine metal particles contained in a specific dispersing agent, and drying the dispersing agent applied to the surfaces of said opposing electrodes so that said fine metal particles are immobilized to the surfaces of said opposing electrodes. By coating the electrode surfaces with fine metal particles, it is possible to increase the capacitor area, so that the sensitivity of the sensor can be improved.

Preferably, the material of said fine metal particles is a material selected from a group consisting of gold, silver, platinum and copper. Immobilization of the probe DNAs is facilitated by the utilization of such materials.

Preferably, the method includes the step of applying said fine metal particles as a coating after an insulating film that partitions said pair of opposing electrodes is formed. By forming such an insulating film, the electrical continuity between the electrodes can be effectively prevented even if the fine metal particles are applied as a coating; furthermore, the distance between the electrodes can be narrowed, so that the capacitance of the capacitor can be increased, thus making it possible to increase the sensitivity of the sensor.

Preferably, the material of said insulating film is polyimide. The surface treatment is facilitated by using polyimide.

Preferably, the method includes a surface treatment step in which the surfaces of said opposing electrodes are endowed with an affinity for liquids, and the surface of said insulating film is endowed with liquid-repellent property. As a result of such a surface treatment step, electrical continuity between the electrodes caused by the deposition of fine metal particles contained in the dispersing agent on the insulating film can be effectively prevented.

Preferably, said surface treatment step is a low pressure plasma treatment performed in a reduced-pressure atmosphere, or an atmospheric-pressure plasma treatment performed in an atmospheric-pressure atmosphere, using oxygen gas containing fluorine or fluorine compounds. As a result of such a surface treatment, the insulating film can be endowed with liquid-repellent property, and the electrode surfaces can be endowed with an affinity for liquids.

Preferably, the method includes the step in which a biologically recognizable molecules that selectively react with specific biomolecules, e. g., probe DNAs, are immobilized as receptors to the surfaces of said fine metal particles. As a result, a DNA micro-array can be realized.

The biological reaction detection method of the present invention uses biologically recognizable molecules that react selectively with specific biomolecules as receptors, and comprises a liquid jetting step in which a sample solution containing said specific biomolecules is discharged by liquid jetting head into a reaction well in which a pair of opposing electrodes having said receptors immobilized on their surfaces is formed, and a detection step in which said reaction is detected by converting the amount of the capacitance variation of the capacitance element consisting of said pair of opposing electrodes into an electrical signal. Since the reaction well is filled with the sample solution by means of a liquid jetting head, accurate control is possible when the liquid is discharged into an extremely small spot.

Preferably, in said detection step, said reaction is detected on the basis of the amount of the current variation that is output from the current output terminal of a transistor whose current-controlling terminal is connected to one of said pair of opposing electrodes. The detection signals from the sensor cell can be converted into numerical values and subjected to data processing by a computer, so that this method is suitable for large-scale genetic analysis.

The genetic analytical method of the present invention comprises the steps of discharging a sample solution containing target DNAs by a liquid jetting head into reaction wells in which a capacitance element consisting of a pair of comb-shaped opposing electrodes having probe DNAs immobilized to their surfaces is formed, detecting the capacitance variation of said capacitance elements caused by the DNA hybridization inside said reaction wells from the amount of output current variation that is output from the drain terminal of a field effect transistor whose gate terminal is connected to any of said pair of comb-shaped opposing electrodes, and performing genetic analysis by subjecting the values of the output currents that are output from a plurality of reaction wells to data analysis by a computer. Genetic analysis can be performed easily and economically by means of such a method.

Preferably, said reaction wells are formed inside sensor cells that are arranged into a matrix, and the probe DNAs immobilized inside adjacent sensor cells are prepared so that their base sequences are slightly different each other. Since the DNA hybridization can take place even if the base sequences are not quite complementary, the base sequence of the target DNA can be inferred from the distribution of the output currents of the sensor cells if the base sequences of the probe DNAs are prepared slightly different each other between adjacent sensor cells.

### BRIEF DESCRIPTION OF THE DRAWINGS

Fig. 1 is a structural diagram of main circuits of a bio-sensor according to Embodiment 1;
Fig. 2 is a structural diagram of main circuits of a bio-sensor according to Embodiment 4;
Fig. 3 is a plan view of a pair of opposing electrodes according to Embodiment 1;
Fig. 4 is a cross-sectional view of the opposing electrodes shown in Fig. 3;
Fig. 5 is a plan view of a pair of opposing electrodes according to Embodiment 2;
Fig. 6 is a cross-sectional view of the opposing electrodes shown in Fig. 5;
Fig. 7A is a cross-sectional view of the film formation process of the comb-shaped opposing electrodes according to Embodiment 2;
Fig. 7B is a cross-sectional view of the film formation process of the insulating film according to Embodiment 2;
Fig. 7C is a cross-sectional view of the process whereby the electrodes are coated with fine metal particles according to Embodiment 2;
Fig. 7D is a cross-sectional view of the process whereby fine metal particles are adsorbed on the electrodes according to Embodiment 2;
Fig. 7E is a cross-sectional view of the opposing electrodes to which probe DNA is immobilized according to Embodiment 2;
Fig. 8 is a plan view of a pair of opposing electrodes according to Embodiment 3; and
Fig. 9 is a cross-sectional view of the opposing electrodes shown in Fig. 8.

### DESCRIPTION OF THE PREFERRED EMBODIMENTS

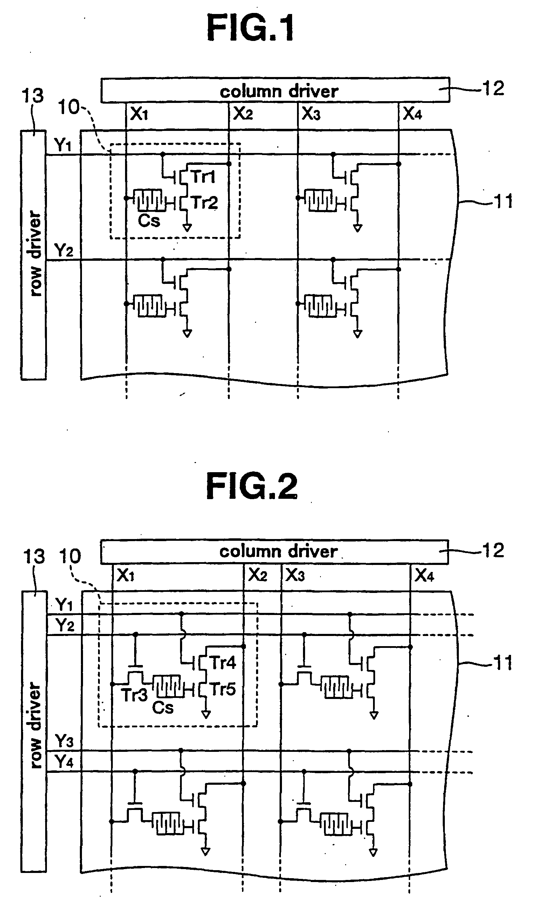

Fig. 1 is a structural diagram of the main circuits of the bio-sensor. This sensor comprises sensor cells 10 which are arranged into a matrix with N rows and M columns on a substrate 11 so as to form a sensor cell matrix, a column driver 12 which drives column-selecting lines (X₁, X₂, ...) used to supply a specific voltage to groups of sensor cells 10 that are lined up in the column direction of said sensor cell matrix, and a row driver 13 which drives row-selecting lines (Y₁, Y₂ ...) used to select groups of sensor cells 10 that are lined up in the row direction of the sensor cell matrix, and to control the switching of the sensing function in the sensor cells 10. The sensor cells 10 are sensors that are used to detect the hybridization of the probe DNA and target DNA inside the reactions wells as an electrical signal; each of these sensor cells 10 comprises a capacitor Cs which detects the DNA hybridization on the basis of the capacitance variation, a switching transistor Tr1 which controls the switching of the sensing function of the sensor cell 10, and a transistor Tr2 used as a transducer (signal converting element) which converts the hybridization of the probe DNA and target DNA inside the reaction well into an electrical signal. The switching transistor Tr1 and transistor Tr2 are field effect transistors (MOSFETs).

The capacitor Cs is formed inside a reaction well as a depression in the substrate 11, and is constituted of a pair of opposing electrodes. Probe DNAs are immobilized at a high density to each of the electrodes, and target DNAs with complementary base sequences to the probe DNAs hybridizes when the reaction well is filled with a sample solution containing these target DNAs. Accordingly, a sensor which has a high specificity to a determined base sequence can be realized. Since the base sequences of the probe DNAs immobilized in the individual sensor cells 10 are known in advance, the base sequence of the target DNA can be determined from the distribution of the target DNAs that are hybridized.

Here, an outline of the operating principle of the sensor cells 10 will be described. In order to place the sensor cell 10 shown in Fig. 1 in an active state, and output the sensing results as an electrical signal, the row-selecting line Y₁ is set at the H level, so that the switching transistor Tr1 is caused to shift from an "off" state (closed state) to an "on" state (open state); meanwhile, the column-selecting lines X₁ and X₂ are set at the H level respectively. Since the switching transistor Tr1 is in an "on" state, the power supply voltage from the column-selecting fine X₂ is supplied to the source terminal of the transistor Tr2 via the switching transistor Tr1, so that the transistor Tr2 is in a state that allows operation in the pinch-off region. As a result of the operation of the transistor Tr2 in the pinch-off region, the transistor Tr2 outputs a drain current that is extremely stable with respect to temperature variations and fluctuations in the power supply voltage that is supplied from the column-selecting line X₂; accordingly, the circuit is electrically equivalent to a constant current source. The current value that is output from this constant current source is uniquely determined according to the gate voltage of the transistor Tr2.

Meanwhile, when hybridization of the DNA fragments occurs in the reaction well, double-stranded DNA is formed by complementary binding, so that the dielectric constant and inter-electrode distance of the capacitor Cs change. The capacitance value of the capacitor Cs is proportional to the dielectric constant and inversely proportional to the inter-electrode distance; thus, the capacitance value of the capacitor Cs is varied due to said hybridization. Since the power supply voltage that is applied to the gate terminal of the transistor Tr2 via the column-selecting line X₁ is determined by the capacitance value of the capacitor Cs, the mutual conductance of the transistor Tr2 also varies when capacitance value of the capacitor Cs varies. The hybridization of the DNA in the reaction well can be monitored in real time by reading the value of the output current from the sensor cell 10 before and after hybridization.

Thus, genetic analysis can be performed in real time by spotting probe DNAs that have slightly different base sequences each other at a high density in the individual sensor cells 10 that is arranged into a matrix, inputting the output signals from the individual sensor cells 10 into a computer, and converting said output signals into numerical values and subjecting these values to data analysis in this computer. It may be predicted that the base sequence of the target DNA, immobilized in the sensor cell 10 that shows the greatest amount of the output current variation, has the highest homology (genetic similarity) to the probe DNA. The genetic analytical technology of the present embodiment can be used in the investigation of genetic diseases, or in forensic medical investigations or the like. Furthermore, since the sensor cells 10 function as capacitance type sensors, the hybridization of DNA in the reaction wells can be detected with high sensitivity, so that this system is superior in terms of quick signal detection. Moreover, in said construction, the column-selecting lines may also be called "voltage supply lines", the row-selecting lines may also be called "scanning lines", the column driver may also be called an "X driver", and the row driver may also be called a "Y driver". Furthermore, the bio-sensor of the present embodiment may also be called a "bio-chip", "DNA chip" or "DNA micro-array".

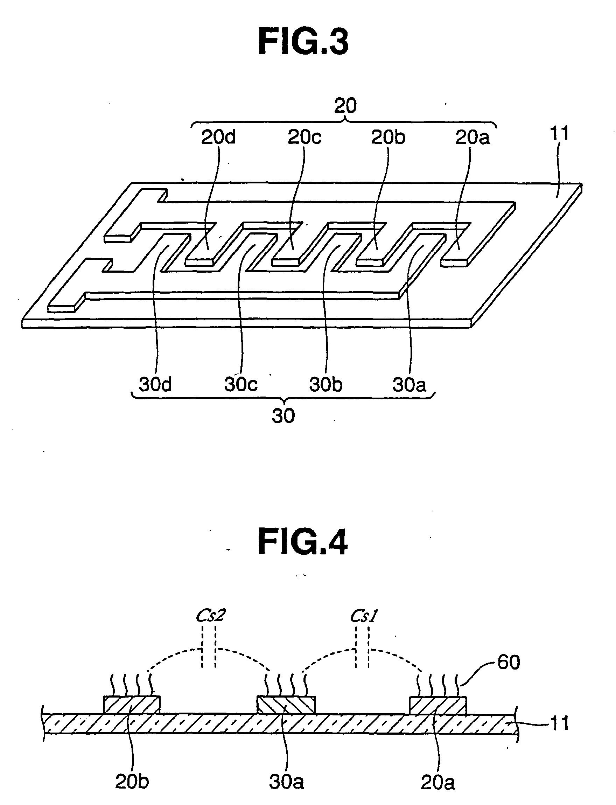

Fig. 3 is an external perspective view of the capacitor Cs. The capacitor Cs is constituted of a comb electrode 20 and a comb electrode 30 that are formed on a substrate 11. The comb electrode 20 and comb electrode 30 are formed in a comb shape so that these electrodes engage with each other, and the electrodes are disposed facing each other with a slight gap left between the electrodes. The comb electrode 20 comprises comb parts 20a through 20d which are formed substantially parallel to each other at substantially same intervals, while the comb electrode 30 comprises comb parts 30a through 30d which are disposed substantially parallel to each other at substantially same intervals so that these comb parts are disposed on either side of the respective comb parts 20a through 20d. As a result of such a structure, it is possible to maximize the capacitor area; accordingly, the amount of the output current variation that is accompanied by the capacitance variation can be increased, so that the sensing sensitivity can be improved. Gold, silver, platinum, copper, aluminum or the like can be used as the electrode material of said comb electrode 20 and comb electrode 30, and a method suited to the film-forming material used can be selected from sputtering methods, plating methods, CVD methods and the like as the film forming method of these electrode materials. For example, in cases where a sputtering method is used, a procedure may be adopted in which the surface of the substrate 11 is coated with a resist and baked, exposure and development are then performed via a metal mask corresponding to the comb electrode pattern, a sputtered film is then formed over the entire surface of the resist, and the resist is then stripped away. The electrode material that is used may be determined in accordance with the means used to immobilize the probe DNAs and the like. For example, in cases where the probe DNAs are immobilized to the surfaces of the comb electrode 20 and comb electrode 30 via gold-sulfur coordinate bonds, it is desirable to use gold, silver, platinum or copper. Furthermore, a glass substrate, plastic substrate, silicon substrate or the like can be used as the substrate 11.

The capacitor Cs shown in Fig. 3 shows only the bottom part of the reaction well. This reaction well is surrounded by an insulating film such as a silicon oxide film or the like, and is constructed so that the reaction well can be filled with a specific volume of a sample solution containing the target DNAs. Furthermore, since the reaction well has a size of several micrometers, it is desirable to use a liquid jetting head (ink jet head) to fill the reaction well with said sample solution containing the target DNAs. If a liquid jetting head is used, accurate control of the discharge of liquid droplets into a very small spot can be realized. Furthermore, in cases where there is a source of electrical noise near the sensor in a volume type sensor of this sort, the effect of electrical noise caused by stray capacitance is large; accordingly, it is desirable to use a structure that minimizes stray capacitance.

Fig. 4 is a cross-sectional view of the capacitor Cs shown in Fig. 3; this figure shows only the parts of the comb part 20a, comb part 30a and comb part 20b. The comb electrode 20 and comb electrode 30 are constructed from gold thin films that are patterned in a comb shape, and probe DNAs 60 are bonded to the surfaces of these electrodes via gold-sulfur bonds with thiol groups introduced at the ends of the DNAs. A method for introducing thiol groups to oligonucleotides is disclosed in detail in Chemistry Letters 1805-1808 (1994) or Nucleic Acids Res., 13, 4484 (1985). DNAs which have a base sequence that is complementary to that of the target DNA, e. g., a single-stranded DNA cut by restriction enzymes from DNA extracted from biological samples and purified by electrophoresis or the like, or biochemically synthesized oligonucleotides, PCR (polymerase chain reaction) products, cDNA or the like, can be used as the DNAs as probe DNAs 60. Meanwhile, DNA obtained by using a gene-decomposing enzyme or ultrasonic treatment to decompose DNA chains extracted from biological materials, single-stranded DNA amplified by PCR from specific DNA chains or the like can be used as the target DNA.

As is shown in the same figure, a very small capacitor Cs1 is formed between the comb part 20a and the comb part 30a, and a very small capacitor Cs2 is formed between the comb part 30a and the comb part 20b. Very small capacitors are also formed between the other comb parts not shown in the figure, and the capacitance values of these individual very small capacitors are caused to vary by the hybridization of the probe DNA 60 and target DNA, so that the sum of these variations is expressed as a variation in the capacitance of the capacitor Cs. The variation in the capacitance of the capacitor Cs is detected as a variation in the output current of the sensor cell 10.

In the description above, a case in which genetic analysis was performed by using probe DNAs as the receptors of the bio-sensor was described as an example. However, the present invention is not limited to this; for example, antigen-antibody reactions can be detected by using antigens as the receptors, and enzyme-substrate reactions can be detected by using enzymes as the receptors. In other words, various types of biochemical substances can be sensed by appropriately selecting a biomolecule that can recognize a specific molecule as the receptor in accordance with the application of the bio-sensor. Such a bio-sensor can be applied to point-of-care devices and health care devices used in medical facilities or by individuals.

In the present embodiment, a large capacitor area can be ensured by using comb electrodes, so that the sensitivity of the sensor can be increased. Furthermore, since a capacitor Cs with a relatively large capacitance can be formed in a very small area, the degree of integration of the sensor cells can be increased, so that large quantities of genetic information can be analyzed at one time. In addition, the hybridization of the probe DNA and target DNA can be detected as a converted electrical signal, so that the reaction can be suitably monitored in real time. Furthermore, there is no need to observe a fluorescent reaction of target DNA labeled with a fluorescent dye as in conventional methods, so that the construction of the apparatus can be simplified. Moreover, since the output signals from the respective sensor cells can be converted into numerical values and subjected to data processing by a computer, this method is suitable for use in large-scale genetic analysis. Furthermore, since the reaction wells have a size of several micrometers on a side, the sensor array can be highly integrated, so that the volume of sample solution used in genetic analysis and the like can be reduced, thus making it possible to increase the reaction efficiency. Moreover, since the sample solution can be introduced using a liquid jetting head, accurate liquid droplet discharge control is possible.

### Embodiment 2 of the Invention

The present embodiment will be described below with reference to respective figures.

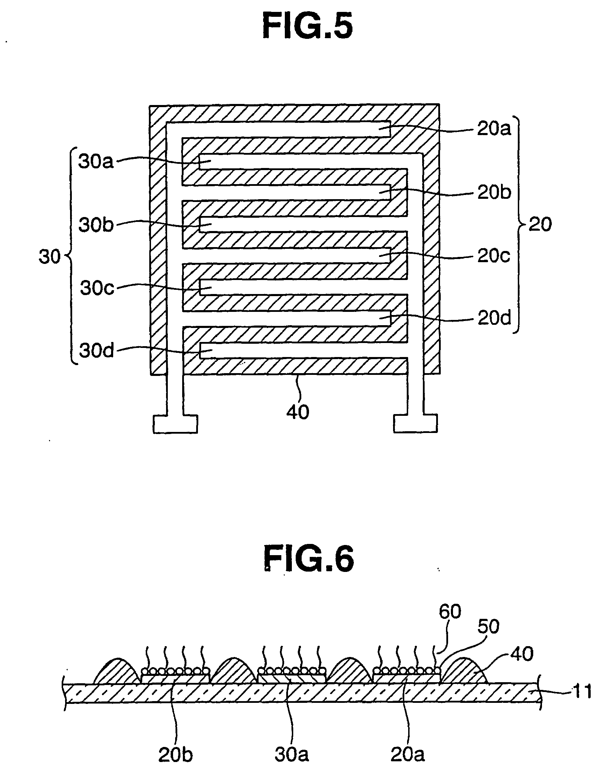

Fig. 5 is a plan view of a second embodiment of the capacitor Cs. In the present embodiment, the distance between the opposing electrodes is minimized by forming an insulating film 40 between a comb electrode 20 and comb electrode 30 that face each other across a very small gap. Thus, since the inter-electrode distance is narrowed, the capacitance of the capacitor can be increased, so that the sensitivity of the sensor can be increased. The insulating film 40 electrically separates the comb electrode 20 and comb electrode 30 so that current path (short-circuit) between these electrodes is prevented. This insulating film 40 may also be called a "separating wall", "partition", "separating member", "partitioning member" or "dividing member". For example, an organic insulating material such as a polyimide or the like is suitable for use as the material of the insulating film 40. As will be described later, if a surface treatment is performed in cases where a polyimide is used, the affinity for liquid droplets supplied from the outside (liquid affinity) in the manufacturing process of the capacitor Cs can easily be controlled.

Fig. 6 is a cross-sectional view of the capacitor Cs shown in Fig. 5. This figure shows how fine metal particles 50 are deposited on the surfaces of the comb electrode 20 and comb electrode 30. By increasing the surface area (capacitor area) of the comb electrode 20 and comb electrode 30, these fine metal particles 50 ensure a maximum capacitance of the capacitor, so that the sensitivity of the sensor can be increased. In cases where fine gold particles are used as the fine metal particles 50, the probe DNA 60 can be bonded to the surfaces of the fine metal particles 50 via gold-sulfur coordinate bonds. Other suitable materials that can be used as the fine metal particles 50 include silver, platinum, copper and the like.

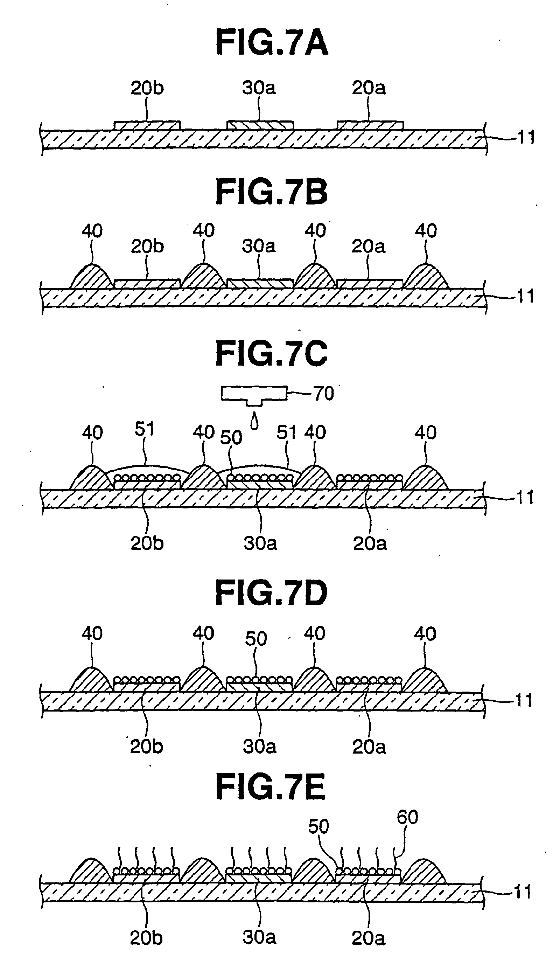

Figs. 7A through 7E are cross-sectional views illustrating the manufacturing process of the capacitor Cs. As is shown in Fig. 7A, a comb electrode 20 and comb electrode 30 are formed in a comb pattern on the surface of a substrate 11. Examples of film formation methods that can be used to form these electrodes include sputtering methods, plating methods, CVD methods and the like. Next, as is shown in Fig. 7B, an insulating film 40 is formed so that the gaps between the comb electrode 20 and comb electrode 30 are filled. The formation of the insulating film 40 can be accomplished using any desired method such as a lithographic method, printing method, ink jet method or the like. For example, in cases where a lithographic method is used, an insulating organic material is applied as a coating by a method such as spin coating, spray coating, roll coating, die coating, dip coating or the like, and this film is then coated with a resist. Then, masking is performed in accordance with the pattern shapes of the comb electrode 20 and comb electrode 30, the resist is exposed and developed, and the resist conforming to the shape of the insulating film 40 is allowed to remain. Finally, etching is performed to remove the organic insulating material without being masked. In cases where a printing method is used, an organic insulating material can be directly applied by any desired method such as relief printing, flat-plate printing, intaglio printing or the like so that the gaps between the comb electrode 20 and comb electrode 30 are filled. In cases where an ink jet method is used, an organic insulating material can be applied so that the gaps between the comb electrode 20 and comb electrode 30 are filled, and can then be dried under appropriate temperature conditions.

Next, as is shown in Fig. 7C, fine metal particle 50 are dispersed in an appropriate dispersing agent 51, and these fine metal particle 50 are applied to the comb electrode 20 and comb electrode 30 using a liquid jetting head 70. There are no particular restrictions on the dispersing agent 51 that is used, as long as this agent is a medium that allows the stable discharge of liquid droplets. An agent with physical properties that allow the discharge of liquid droplets in a state in which the fine metal particles 50 and dispersing agent 51 are mixed is suitable. For example, high-melting-point organic solvents such as xylene, toluene, dodecylbenzene, mineral spirit, tridecane, α-terpineol and the like can be used, with these solvents being adjusted so that the viscosity is 1 cPs to 20 cPs and the surface tension is 30 mN/m to 50 mN/m. In order to achieve the stable discharge of liquid droplets from the liquid jetting head 70, it is desirable that the material be a slow-drying material, and it is desirable to select a material with a high melting point.

In the jetting process of the fine metal particles 50, the fine metal particles 50 that are deposited on the comb electrode 20 and comb electrode 30 may also be laminated in several layers; however, it is desirable that the particles be appropriately dispersed so that there is no variation among individual sensor cells. Either a thermal jet type/Bubble Jet (registered trademark) type head which discharges liquid droplets by using thermal energy to generated gas bubbles, or a piezo-jet type head which discharges liquid droplets by converting electrical energy into mechanical energy, may be used as the liquid jetting head 70. Next, as is shown in Fig. 7D, the dispersing agent 51 is dried under appropriate temperature conditions, so that the fine metal particles 50 are adsorbed on the surfaces of the comb electrode 20 and comb electrode 30. Finally, the capacitor Cs is completed by bonding the probe DNAs 60 to the fine metal particles 50 (Fig. 7E).

Furthermore, in the discharging process of the fine metal particles 50 shown in Fig. 7C, if the fine metal particles 50 are deposited on the insulating film 40 as a result of the insulating film 40 being coated with the fine metal particles 50 that are discharged from the liquid jetting head 70, and if the comb electrode 20 and comb electrode 30 are placed in an electrically continuous state, the device will become unable to function as a capacitor. Accordingly, it is desirable to perform a surface treatment so that the surfaces of the comb electrode 20 and comb electrode 30 are endowed with an affinity for liquids, and so that the surface of the insulating film 40 is endowed with liquid-repellent properties. For example, a low pressure plasma treatment or atmospheric-pressure plasma treatment in which plasma irradiation is performed in a reduced-pressure atmosphere or atmospheric-pressure atmosphere using oxygen gas containing fluorine or fluorine compounds can be performed as such a surface treatment. If this is done, unreacted groups will be generated by plasma discharge on the surfaces of inorganic materials such as those of the comb electrode 20 and comb electrode 30, and these unreacted groups will be oxidized by said oxygen so that polar groups such as carbonyl groups, hydroxyl groups or the like are generated. Polar groups show an affinity for liquids containing polar molecules such as water or the like, and show a non-affinity for liquids containing non-polar molecules.

Meanwhile, in parallel with said reaction, a phenomenon whereby fluorine compound molecules enter the surface of the organic material also occurs on the surface of the insulating film 40 (which consists of an organic insulating material), so that the surface is converted into a non-polar state. As a result, this surface shows a non-affinity for liquid that contain polar molecules such as water or the like, and shows an affinity for liquids that contain non-polar molecules. As fluorine or fluorine compounds contained in oxygen, halogen gases such as CF₄, CF₆, CHF₃ and the like are suitable. In regard to the degree of affinity for the dispersing agent 51, a contact angle of less than 20 degrees is desirable for the surfaces of the comb electrode 20 and comb electrode 30, and a contact angle of 50 degrees or greater is desirable for the surface of the insulating film 40.

In the present embodiment, an insulating film 40 is formed between the pair of comb electrodes that constitute the capacitor Cs, so that the inter-electrode distance is minimized; accordingly, the capacitance of the capacitor Cs can be increased, so that the sensitivity of the sensor can be increased. Furthermore, since fine metal particles that are used to increase the capacitor area are deposited on the surfaces of the comb electrodes, the capacitor area can be increased, so that the sensitivity of the sensor can be increased. Moreover, as a result of the installation of said insulating film 40, electrical continuity between the electrodes can be prevented even in cases where the fine metal particles 50 that are discharged from the liquid jetting head 70 overflow from the surfaces of the comb electrodes. Furthermore, since the affinity of the surfaces of the comb electrodes and the surface of the insulating film for the liquid droplets that are discharged from the liquid jetting head 70 (in particular, the dispersing agent containing fine metal particles) is controlled by a surface treatment such as an atmospheric-pressure plasma treatment, electrical continuity between the comb electrodes caused by the deposition of fine metal particles 50 on the surface of the insulating film 40 can be prevented.

### Embodiment 3 of the Invention

The present embodiment will be described below with reference to respective figures.

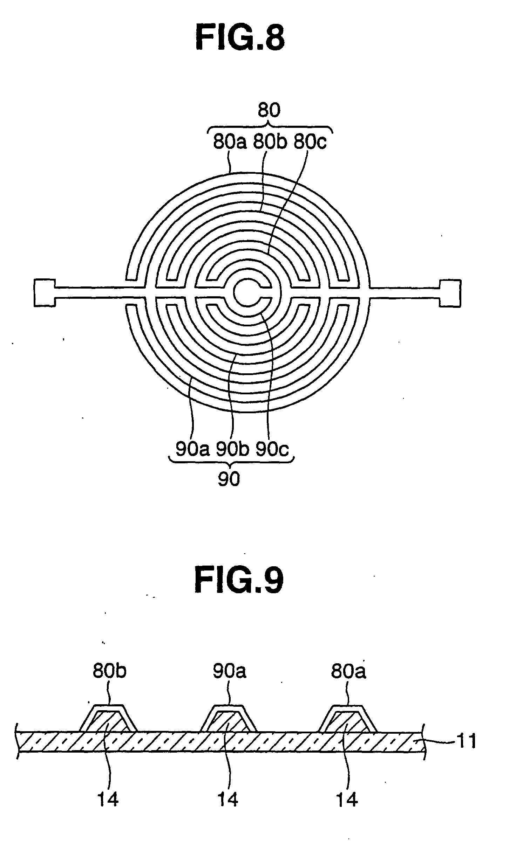

Fig. 8 is a plan view of a third embodiment of the capacitor Cs. In the present embodiment, the capacitor Cs is constituted of a pair of opposing electrodes (electrodes 80 and 90) that are formed into a circular arc shape. The electrode 80 comprises electrodes 80a, 80b and 80c which are formed substantially in the shape of concentric circles with different internal diameters. Meanwhile, the electrode 90 comprises electrodes 90a, 90b and 90c which are formed substantially in the shape of concentric circles with different internal diameters. In the case of these electrodes, cathode and anode are alternately disposed, so that very small capacitors are respectively formed between the inner circumferential surface of the electrode part 80a and the outer circumferential surface of the electrode part 90a, between the inner circumferential surface of the electrode part 90a and the outer circumferential surface of the electrode part 80b, ..., and between the inner circumferential surface of the electrode part 80c and the outer circumferential surface of the electrode part 90c. The sum of the capacitance values of these very small capacitors is the capacitance of the capacitor Cs. Fig. 9 shows a sectional view of the same capacitor. The respective electrodes 80a, 90a, 80b, ..., 90c described above consist of metal thin films which are formed so that these films cover an insulating film 14 which is patterned in the form of a circular arc on the surface of a substrate 11. Since these electrodes 80a, 90a, 80b, ..., 90c are formed into a protruding shape, the capacitor area can be increased, thus contributing to an increase in the sensitivity of the sensor. Probe DNAs 60 are immobilized to the surfaces of these electrodes 80a, 90a, 80b, ..., 90c by gold-sulfur coordinate bonds or the like.

Furthermore, in the present embodiment, the sensitivity of the sensor can be increased by forming an insulating film between the respective electrodes 80a, 90a, 80b,...,90c to narrow the inter-electrode distance and to secure the maximum capacitance of the capacitor. Furthermore, the sensitivity of the sensor can be increased by depositing fine metal particles on the surfaces of these electrodes to secure the maximum capacitance of the capacitor. It is desirable that the application of such fine metal particles be accomplished by discharging fine metal particles mixed with a dispersing agent from a liquid jetting head.

### Embodiment 4 of the Invention

The present embodiment will be described below with reference to respective figures.

Fig. 2 is a structural diagram of the main circuits in another embodiment of the bio-sensor. This sensor comprises sensor cells 10 which are arranged into a matrix with N rows and M columns on a substrate 11 so that a sensor cell matrix is formed, a column driver 12 which drives column-selecting lines X₁, X₂, ... that are used to supply a specific voltage to a group of sensor cells 10 that are lined up in the column direction of said sensor cell matrix, and a row driver 13 which drives row-selecting lines Y₁, Y₂, ... that are used to select a group of sensor cells 10 that are lined up in the row direction of the sensor cell matrix, and to control the switching of the sensing function in the sensor cells 10. Each of the sensor cells 10 comprises a capacitor Cs which is constituted of a pair of electrodes and which detects the hybridization of DNA inside a reaction well, switching transistors Tr3 and Tr4 which are used to control the switching of the sensing function of the sensor cell 10, and a transistor Tr5 which is used as a transducer that converts the DNA hybridization into an electrical signal. The construction of the capacitor Cs may be any of the constructions of Embodiments 1 through 3.

Here, an outline of the operating principle of each sensor cell 10 will be described. In the same figure, in order to place the sensor cell 10 in an active state and output the sensing results as an electrical signal, the row-selecting lines Y₁ and Y₂ are respectively set at the H level, and the switching transistors Tr3 and Tr4 are shifted from an "off" state to an "on" state, while the column-selecting lines X₁ and X₂ are also set at the H level. Since the switching transistor Tr4 is in an "on" state, the power supply voltage from the column-selecting line X₂ is supplied to the source terminal of the transistor Tr5 via the switching transistor Tr4, so that the transistor Tr5 can operate in the pinch-off region. As a result of the transistor Tr5 operating in the pinch-off region, a drain current that is extremely stable with respect to temperature variations and fluctuations in the power supply voltage supplied from the column-selecting line X₂ is output, so that this circuit is electrically equivalent to a constant current source. The current value that is output from this constant current source is uniquely determined by the gate voltage of the transistor Tr5.

Meanwhile, since the switching transistor Tr3 is in an "on" state, the power supply voltage that is supplied from the column-selecting line X₁ is transmitted to the gate terminal of the transistor Tr5 via the capacitor Cs. Since the voltage that is applied to gate terminal of the transistor Tr5 is determined by the capacitance value of the capacitor Cs, variations in the capacitance of the capacitor Cs can be detected as variations in the drain current of the transistor Tr5. If the present embodiment is used, detection signals from the respective sensor cells can be converted into numerical values and subjected to data processing by a computer; accordingly, this system is suitable for use in large-scale genetic analysis.

According to the present invention, biological reactions can be detected as electrical signals. Accordingly, real time monitoring is possible, and detection signals from respective sensor cells can be converted into numerical values and subjected to data processing by a computer, so that this system is suitable for use in large-scale genetic analysis. Furthermore, there is no need to observe the fluorescent reaction of target DNA labeled with a fluorescent dye as in conventional methods, so that the construction of the apparatus can be simplified. Moreover, by using comb electrodes, it is possible to secure a large capacitor area, so that the sensitivity of the sensor can be increased. Furthermore, by depositing fine metal particles on the surfaces of the pair of opposing electrodes, it is possible to increase the capacitor area, so that the sensitivity of the sensor can be increased.

Moreover, by forming an insulating film between the pair of opposing electrodes, it is possible to prevent electrical continuity between the electrodes even in cases where the fine metal particles that are discharged from the liquid jetting head overflow from the surfaces of the electrodes. Furthermore, since the affinity of the surfaces of the electrodes and the surface of the insulating film for the liquid droplets that are discharged from the liquid jetting head can be controlled by means of a surface treatment such as an atmospheric-pressure plasma treatment or the like, electrical continuity between the electrodes caused by the deposition of fine metal particles on the insulating film can be prevented.

Furthermore, since the reaction wells have a size of several micrometers on a side, a high degree of integration of the sensor array can be realized, and the volume of the sample solution used for genetic analysis and the like can be reduced, which is economical. Moreover, since the reaction wells can be filled with the sample solution by means of a liquid jetting head, accurate liquid droplet discharge control is possible.

## Claims

1. A sensor cell comprising:
a capacitance element which consists of a pair of opposing electrodes having receptors immobilized to their surfaces, wherein said receptors are biologically recognizable molecules that react selectively with specific biomolecules; and
a transducer which outputs as an electrical signal the amount of the capacitance variation of the said capacitance element, wherein said capacitance variation is caused by the reaction between said receptors and biomolecules.

2. The sensor cell according to claim 1, wherein each of said pair of opposing electrodes is a comb electrode formed into a comb shape.

3. The sensor cell according to claim 1, wherein each of said pair of opposing electrodes has a plurality of circular-arc-form electrode parts with different internal diameters, and forms a circular-arc-form capacitor between the facing electrode parts.

4. The sensor cell according to claim 1, wherein an insulating film is formed between said pair of opposing electrodes to partition the respective electrodes.

5. The sensor cell according to claim 1, wherein fine metal particles are deposited on the surfaces of said electrodes.

6. The sensor cell according to claim 5, wherein the material of said fine metal particles is selected from a group consisting of gold, silver, platinum and copper.

7. The sensor cell according to claim 1, wherein the material of said pair of opposing electrodes is selected from a group consisting of gold, silver, platinum, copper and aluminum.

8. The sensor cell according to claim 1, wherein said pair of opposing electrodes is formed inside a reaction well.

9. The sensor cell according to claim 1, wherein said transducer is a field effect transistor which varies the mutual conductance in accordance with the capacitance variation of said capacitance element.

10. The sensor cell according to claim 1, wherein said biologically recognizable molecules are probe DNAs.

11. A bio-sensor comprising:
a sensor cell matrix in which sensor cells that output biological reactions as electrical signals are arranged into a matrix;
a row driver which supplies a specific voltage signal to row-selecting lines connected to a group of sensor cells that are lined up in the row direction of said sensor cell matrix; and
a column driver which supplies a specific voltage signal to column-selecting lines connected to a group of sensor cells that are lined up in the column direction of said sensor cell matrix;
wherein each of said sensor cells comprises a capacitance element which consists of a pair of opposing electrodes having said receptors immobilized to the electrode surfaces, wherein said receptors are biologically recognizable molecules that react selectively with specific biomolecules; a transistor whose current-controlling terminal is connected to said capacitance element so that this transistor varies the current value that is output from the current output terminal in accordance with the amount of the capacitance variation of said capacitance element, wherein said capacitance variation is caused by the reaction between said receptors and biomolecules; and a switching element which supplies the voltage signal supplied from said column driver to the current input terminal of said transistor; and
said switching element is placed in an open state by a voltage signal supplied from the row driver via said row-selecting line, so that the voltage signal supplied from said column driver via said column-selecting line is input into the current input terminal of said transistor.

12. The bio-sensor according to claim 11, wherein each of said pair of opposing electrodes is a comb electrode formed into a comb shape.

13. The bio-sensor according to claim 11, wherein each of said pair of opposing electrodes has a plurality of circular-arc-form electrode parts with different internal diameters, and forms a circular-arc-form capacitor between the facing electrode parts.

14. The bio-sensor according to claim 11, wherein an insulating film to partition the respective electrodes is formed between said pair of opposing electrodes.

15. The bio-sensor according to claim 11, wherein fine metal particles are deposited on the surfaces of said electrodes.

16. The bio-sensor according to claim 15, wherein the material of said fine metal particles is selected from a group consisting of gold, silver, platinum and copper.

17. The bio-sensor according to claim 11, wherein the material of said pair of opposing electrodes is selected from a group consisting of gold, silver, platinum, copper and aluminum.

18. The bio-sensor according to claim 11, wherein said pair of opposing electrodes are formed inside a reaction well.

19. The bio-sensor according to clam 11, wherein said transistor is a field effect transistor, said current-controlling terminal is the gate terminal of the transistor, said current input terminal is the source terminal of the transistor, and said current output terminal is the drain terminal of the transistor.

20. The bio-sensor according to claim 11, wherein said biologically recognizable molecules are probe DNAs.

21. A method for manufacturing a capacitance element whose capacitance is varied by biological reactions, comprising the steps of:
forming a pair of opposing electrodes on the surface of an insulating substrate;
coating the surfaces of said pair of opposing electrodes with fine metal particles contained in a specific dispersing agent; and
drying the dispersing agent applied as a coating to the surfaces of said opposing electrodes, so that said fine metal particles are immobilized to the surfaces of said opposing electrodes.

22. The capacitance element manufacturing method according to claim 21, wherein the material of said fine metal particles is selected from a group consisting of gold, silver, platinum and copper.

23. The capacitance element manufacturing method according to claim 21, further comprising a step in which said fine metal particles are applied as a coating after an insulating film that partitions said pair of opposing electrodes has been formed.

24. The capacitance element manufacturing method according to claim 23, wherein the material of said insulating film is a polyimide.

25. The capacitance element manufacturing method according to claim 23, further comprising a surface treatment step in which the surfaces of said opposing electrodes are endowed with an affinity for liquids, and the surface of said insulating film is endowed with liquid-repellent property.

26. The capacitance element manufacturing method according to claim 25, wherein said surface treatment step consists of a low pressure plasma treatment performed in a reduced-pressure atmosphere, or an atmospheric-pressure plasma treatment performed in an atmospheric pressure atmosphere, using oxygen gas containing fluorine or fluorine compounds.

27. The capacitance element manufacturing method according to claim 21, further comprising a step in which biologically recognizable molecules that react selectively with specific biomolecules are immobilized as receptors to the surfaces of said fine metal particles.

28. The capacitance element manufacturing method according to claim 27, wherein said biologically recognizable molecules are probe DNAs.

29. A biological reaction detection method comprising:
a liquid jetting step in which a biologically recognizable molecules that react selectively with specific biomolecules are used as receptors, and a sample solution containing said specific biomolecules is discharged by the liquid jetting head into the reaction well in which a pair of opposing electrodes having said receptors immobilized to their surfaces is formed; and
a detection step in which said reaction is detected by converting the amount of the capacitance variation of the capacitance element consisting of said pair of opposing electrodes into an electrical signal.

30. The biological reaction detection method according to claim 29, wherein said detection step detects said reaction on the basis of the amount of the current variation that is output from the current output terminal of a transistor whose current-controlling terminal is connected to one of said pair of opposing electrodes.

31. The biological reaction detection method according to claim 29, wherein each of said pair of opposing electrodes is a comb electrode formed into a comb shape.

32. The biological reaction detection method according to claim 29, wherein each of said pair of opposing electrodes has a plurality of circular-arc-form electrode parts with different internal diameters, and forms a circular-arc-form capacitor between the facing electrode parts.

33. The biological reaction detection method according to claim 29, wherein an insulating film to partition the respective electrodes is formed between said pair of opposing electrodes.

34. The biological reaction detection method according to claim 29, wherein fine metal particles are deposited on the surfaces of said electrodes.

35. The biological reaction detection method according to claim 34, wherein the material of said fine metal particles is selected from a group consisting of gold, silver, platinum and copper.

36. The biological reaction detection method according to claim 29, wherein said biologically recognizable molecules are probe DNAs.

37. A genetic analytical method comprising the steps of:
discharging a sample solution containing target DNAs by a liquid jetting head into reaction wells in each of which a capacitance element consisting of a pair of comb-shaped opposing electrodes having probe DNAs immobilized to the electrode surfaces is formed;
detecting the amount of the capacitance variation of said capacitance elements caused by the DNA hybridization inside said reaction wells from the amount of the output current variation that is output from the drain terminal of a field effect transistor whose gate terminal is connected to one of said pair of comb-shaped opposing electrodes; and
performing genetic analysis by subjecting the values of the output currents that are output from a plurality of reaction wells to data analysis by a computer.

38. The genetic analytical method according to claim 37, wherein said reaction wells are formed inside sensor cells that are arranged into a matrix, and DNAs immobilized inside adjacent sensor cells are prepared so that their base sequences are slightly different each other.

39. The genetic analytical method according to claim 37, wherein an insulating film is formed between said pair of opposing electrodes.

40. The genetic analytical method according to claim 37, wherein fine metal particles are deposited on the surfaces of said pair of opposing electrodes.

41. The genetic analytical method according to claim 40, wherein the material of said fine metal particles is selected from a group consisting of gold, silver, platinum and copper.
